Europäisches Patentamt

European Patent Office

Office européen des brevets

(11) Numéro de publication: **0 336 857**
**A1**

(12) # DEMANDE DE BREVET EUROPEEN

(21) Numéro de dépôt: **89420108.6**

(22) Date de dépôt: **28.03.89**

(51) Int. Cl.⁴: **A 61 F 13/02**

(30) Priorité: **29.03.88 FR 8804478**

(43) Date de publication de la demande:
**11.10.89 Bulletin 89/41**

(84) Etats contractants désignés:
**AT BE CH DE ES GB GR IT LI LU NL SE**

(71) Demandeur: **MOLINIER S.A.**
**rue des Siccards**
**F-42340 Veauche (FR)**

(72) Inventeur: **Delannoy, Robert**
**3 rue Chernoviz**
**F-75016 Paris (FR)**

(74) Mandataire: **Dupuis, François**
**Cabinet Charras 3 Place de l'Hôtel-de-Ville BP 203**
**F-42005 St. Etienne Cédex 1 (FR)**

(54) **Bande élastique notamment de contention, son procédé de réalisation, et les moyens de mise en oeuvre.**

(57) L'invention se rattache au secteur technique des bandages.

La bande selon l'invention est réalisée à partir d'un support tissé ou tricoté (1) présentant des fils extensibles du type élastomère pour lui conférer une extensibilité positive notamment dans le sens longitudinal de l'ordre de 60 à 100%, ledit support (1) présentant une contexture très aérée, de faible densité et avec une rémanence sensiblement nulle, l'une des faces de ce support recevant un film (2) d'une masse adhésive anallergique.

FIG.1

**EP 0 336 857 A1**

## Description

## Bande élastique, notamment de contention, son procédé de réalisation, et les moyens de mise en oeuvre.

L'invention se rattache au secteur technique des bandages.

Il est connu d'utiliser des bandes de pansement extensibles, dont l'une des faces est traitée pour avoir un coefficient d'adhésivité élevé. De telles bandes trouvent de nombreuses applications, notamment dans les domaines médicaux et orthopédiques et plus particulièrement pour le blocage des artculations et la contention à demeure.

Cependant, ces bandes adhésives présentent certains inconvénients. L'enduction de la masse adhésive a pour effet, dans certains cas, d'obturer la plupart des interstices de la bande de contention qui, dans ces conditions, respire mal. La bande est donc insuffisamment aérée. Or, compte-tenu des applications envisagées pour de telles bandes, l'aération est une caractéristique très importante.

En outre, dans les bandes adhésives connues, notamment celles du type ayant un coefficient d'adhésivité élevé, les masses adhésives employées sont à base de solvant et peuvent induire des phénomène d'allergie ce qui n'est pas compatible avec le but recherché, bien que l'emploi de telles masses adhésives ne soit pas interdit et soit peu réglementé.

L'invention s'est fixée pour but de remédier à ces inconvénients, d'une manière simple, efficace et rationnelle. Pour résoudre le problème posé d'avoir une bande de contention adhésive ayant de bonnes caracéristiques d'aération, ladite bande est réalisée à partir d'un support tissé ou tricoté présentant des fils extensibles du type élastomère pour lui conférer une extensibilité positive notamment dans le sens longitudinal de l'ordre de 60 à 100 %, ledit support présentant une contexture très aérée, de faible densité et avec une rémanence sensiblement nulle, l'une des faces de ce support recevant un film de mass adhésive anallergique.

Avantageusement, ce film est obtenu à partir d'une masse adhésive du type thermofusible.

Le problème posé d'avoir une bande adhésive très aérée, tout en ayant un pouvoir adhésif élevé, est résolu par un procédé particulier de mise en oeuvre. Selon l'invention, le procédé est remarquable par les principales étapes suivantes :
- on porte la masse adhésive à une certaine température que l'on maintient constante jusqu'à une zone d'application sur le support textile,
- on entraîne linéairement le support textile en le maintenant, au niveau de la zone d'application de la masse adhésive, à l'état rétracté avec une tension très réduite, le film de colle étant appliqué sur la face correspondante dudit support, par léchage, au fur et à mesure du défilement du support.

A la sortie de la zone d'enduction de l'adhésif, on applique la face adhésive du support sur un papier silicone.

Pour mettre en oeuvre ce procédé spécifique de fabrication d'une bande adhésive présentant les caractéristiques indiquées, les moyens comprennent essentiellement un poste de reserve de masse adhésive assujetti à des moyens de contrôle et de régulation aptes à la maintenir à une température constante jusqu'à un poste d'enduction monté en combinaison avec des organes de commande aptes à assurer l'entraînement linéaire du support en le maintenant à l'état rétracté, avec une tension réduite, pour rendre adhésive la face correspondante dudit support.

A la sortie du poste d'enduction de la masse adhésive et des organes d'entraînement, la bande enduite coopère avec un poste de réception pour être appliquée et notamment enroulée sur un support notamment du type siliconé.

Les organes de commande et d'entraînement sont composés de deux ensembles de cylindres rotatifs montés de part et d'autres du poste d'enduction, chacun des ensembles comprenant deux cylindres superposés entre lesquels passe le support, les cylindres supérieurs étant montés en pression sur les cylindres inférieurs.

Le poste d'enduction de la masse adhésive comprend des buses d'injection chauffantes reliées à un organe déposeur apte à distribuer la masse adhésive sous forme d'un film d'épaisseur constante, au fur et à mesure de l'avance du support à l'état rétracté.

La vitesse linéaire du support est intégrée pour commander en conséquence le débit de la masse adhésive d'une manière proportionnelle.

L'invention est exposée ci-après plus en détail à l'aide des dessins annexés, dans lesquels :

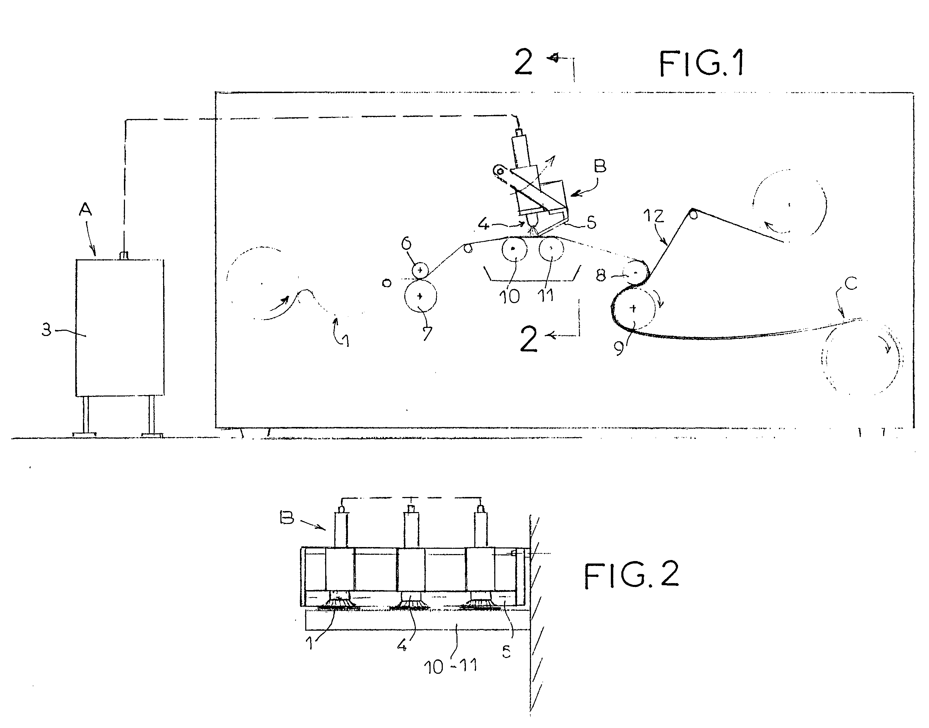

- la figure 1 est une vue de face, à caractère schématique, de la machine montrant les principaux postes pour la réalisation de la bande selon l'invention,
- la figure 2 est une vue en coupe transversale considérée selon la ligne 2.2. de la figure 1,

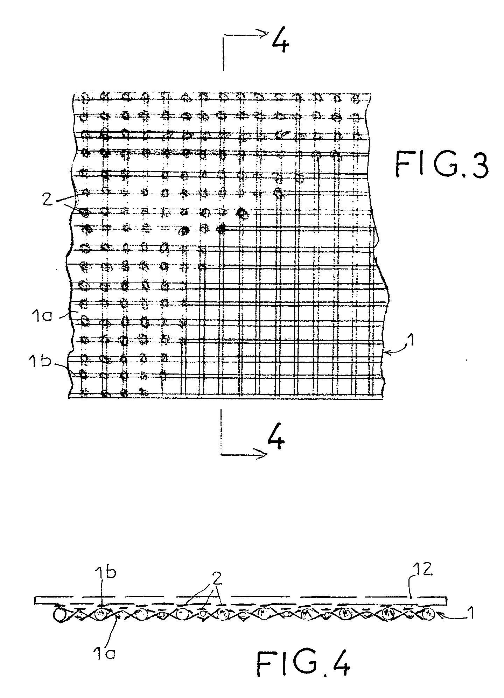

- la figure 3 est une vue en plan à très grande échelle, de la bande montrant la face enduite de la masse adhésive,
- la figure 4 est une vue en coupe transversale considérée selon la ligne 4.4. de la figure 3.

Afin de rendre plus concret l'objet de l'invention, on le décrit maintenant d'une manière non limitative en se référant aux exemples de réalisations des figures des dessins.

La bande est obtenue à partir d'un support tissé ou tricoté (1) présentant, en chaîne notamment, des fils extensibles du type élastomère pour conférer à ladite bande une capacité d'extension dans le sens longitudinal, avec une extensibilité positive de l'ordre de 60 à 100 %. Le support (1) présente une contexture très aérée, de faible densité et avec une rémanence sensiblement nulle.

En outre, la contexture du support est apte à délimiter des parties en creux (1a) et des parties en relief (1b) ré sultant de l'entrelacement des fils de chaîne et des fils de trame. A cet égard, plusieurs modes de tissage ou de tricotage peuvent être prevus.

Quant à la nature des fils et leur disposition dans le support (1), on peut envisager différentes possibilités telles que :
- chaîne composée de fils extensibles et trame en fils non extensibles,
- chaîne et trame en fils extensibles,
- chaîne composée en alternance de fils extensibles et inextensibles, trame en fils inextensibles ou extensibles sans pour cela exclure d'autres combinaisons.

D'une manière importante, selon l'invention l'une des faces du support (1) tel que défini est recouvert d'un film (2) d'une matière adhésive anallergique. Avantageusement, cette matière adhésive est constituée par une colle du type thermofusible.

Il convient maintenant d'analyser comment est appliquée le film (2) sur le support pour obtenir une bande adhésive ayant les caractéristiques recherchées à savoir essentiellement, une aération maximale, un pouvoir collant élevé, et un aspect visuel régulier et uniforme.

Dans ce but, la machine de mise en oeuvre comprend un poste (A) de réserve de masse adhésive, un poste (B) d'enduction et un poste (C) de réception de la bande traitée selon l'invention.

Au poste (A), la masse adhésive (2) est contenue dans un récipient approprié, pour être portée à une certaine température de l'ordre de 140° à 170° C en fonction du produit utilisé.

D'une manière très importante, la masse adhésive est maintenue à une température rigoureusement constante pendant toute la durée du cycle d'enduction, c'est-à-dire depuis le départ, jusqu'à la zone d'enduction située au poste (B). A cet égard, différents capteurs et autres organes de contrôle peuvent être placés le long des conduites d'amenée de la masse adhésive pour assurer la régulation de la température. A noter que la masse adhésive (2) est acheminée du récipient (3) à la zone (B), au moyen de pompes notamment.

Le poste d'enduction en tant que tel, comprend des buses d'injection (4), assujetties à des têtes chauffantes, et reliées à un organe délivreur (5) apte à distribuer la masse adhésive sous forme d'un film d'épaisseur constante. L'ensemble du poste d'enduction (4-5) est escamotable automatiquement à l'arrêt de la machine, au moyen de bras articulés par exemple, pour être en contact ou non avec le support (1) comme indiqué ci-après.

Suivant une autre caractéristique essentielle de l'invention, au niveau du poste d'enduction, la bande ou support (1) préalablement conditionné en rouleau par exemple, coopère avec des moyens aptes à l'entraîner linéairement en la maintenant à l'état rétracté avec une tension réduite. Ces moyens se composent de deux ensembles de cylindres rotatifs (6-7) et (8-9) montés de part et d'autre du dispositif d'enduction (4-5). Chaque ensemble comprend deux cylindres superposés entre lesquels passe le support (1), les cylindres supérieurs (6) et (8) étant montés en pression sur les cylindres inférieurs (7) et (9) qui font office de contre-appui.

Chacun des ensembles de cylindres (6-7) et (8-9) est asservi à des organes de commande pour être entraîné positivement en rotation à une vitesse déterminée parfaitement maîtrisée et réglable, pour entraîner le support (1) selon une tension faible mais rigoureusement constante correspondant à l'état rétracté dudit support (1). Entre les cylindres motorisés (6-7) et (8-9) et sous l'organe racleur et distributeur (5) de la masse adhésive, sont disposés des rouleaux (10 et 11) constituant un plan d'appui horizontal pour le support (1), en vue de l'application du film (2).

A la sortie de l'ensemble des cylindres (8-9), la face encollée (1a) du support (1), est contre-collée notamment sur un papier siliconé (12), engagé entre lesdits cylindres (8) et (9). Le support (1) et le papier (12) sont enroulés par friction à la tension du papier dont l'extensibilité est très sensiblement nulle.

A noter que le cylindre dit d'appel (9), est refroidi pour accélérer d'abaissement de la température de la masse adhésive (2) en évitant ainsi tout risque d'adhérence de cette masse sur le cylindre (9) notamment qui, d'une manière avantageuse, présente un revêtement siliconé ou autre. Ce refroidissement peut s'opérer par la circulation d'un fluide réfrigérant tel que de l'eau envoyé dans le cylindre (9).

Il convient de souligner que les vitesses notamment des rouleaux de déroulage et d'enroulage de la bande, sont asservies par les vitesses nominales des cylindres régulateur (6-7) et (8-9). De même, la vitesse linéaire de la bande est intégrée pour commander en conséquence le débit de la colle d'une manière proportionnelle. Les différentes vitesses sont réglées et affichées en permanence. L'ensemble des moyens de commande est assujetti notamment à une carte à microprocesseur.

Bien évidemment, pendant le cycle d'enduction, l'organe distributeur (5) de la masse adhésive, est mis en contact avec le support (1), en appui sur les rouleaux (10-11). La masse adhésive (2) est envoyée en continu et à débit constant, de sorte que le déplacement linéaire du support (1), au contact de l'organe distributeur (5), assure par léchage, le dépôt du film (2) sur la totalité de la largeur de la face correspondante du support (1).

Compte-tenu de la contexture du support (1), avec des effets de creux et de relief, le film (2) est réparti d'une manière très spécifique, en adhérant en priorité sur les parties en relief dudit support (1). Il en résulte une première rupture du film (2), créant par conséquc.. . une ...ation du support.

En outre, le support (1) étant rendu adhésif à l'état rétracté comme indiqué précédemment, au fur et à mesure de l'étirement dudit support lors de son utilisation, le film (2) est rompu en constituant une pluralité de micro-perforations, conférant une aération optimum à la bande adhésive ainsi exécutée.

A l'arrêt du cycle de fabrication tel que décrit, la pompe d'alimentation de la masse adhésive est stoppée, tandis qui l'ensemble d'enduction (4-5) est relevé instantanément.

Les avantages ressortent bien de la description. En particulier, on souligne et on rappelle :
- la bande adhésive obtenue est très aérée avec un effet visuel parfait,
- l'application d'une masse adhesive du type thermofusible anallergique.

On prévoit également, selon l'invention, de superposer des supports complémentaires après avoir déposé la masse adhésive de manière à constituer un ensemble composite dont l'un présente dans le sens longitudinal une réserve non rendue adhésive. Cette opération peut s'effectuer entre le papier siliconé et la masse adhésive, au moment de l'enroulement du support.

**Revendications**

-1- Bande élastique notamment de contention, réalisée à partir d'un support tissé ou tricoté (1) présentant des fils extensibles du type élastomère pour lui conférer une extensibilité positive notamment dans le sens longitudinal de l'ordre de 60 à 100 %, ledit support (1) présentant une contexture très aérée, de faible densité et avec une rémanence sensiblement nulle, l'une des faces de ce support recevant un film (2) d'une masse adhésive anallergique.

-2- Bande selon la revendication 1, caracérisée en ce que la masse adhésive (2) est une colle du type thermofusible.

-3- Procédé de réalisation d'une bande selon la revendication 1, caractérise par les étapes suivantes :
- on porte la masse adhésive (2) à une certaine température que l'on maintient constante jusqu'à une zone d'enduction sur le support textile (1),
- on entraîne linéairement le support textile en le maintenant, au niveau de la zone d'application de la masse adhésive, à l'état rétracté avec une tension très réduite, le film étant appliqué sur la face correspondante dudit support, par léchage, au fur et à mesure du défilement du support.

-4- Procédé selon la revendication 3, caractérisé en ce qu'à la sortie de la zone d'enduction, on applique la face rendue adhésive du support sur un papier silicone.

-5- Moyens de mise en oeuvre du procédé selon l'une quelconque des revendications 3 et 4, caractérisés en ce qu'ils comprennent essentiellement, un poste (A) de réserve de la masse adhésive assujetti à des moyens de contrôle et de régulation aptes à maintenir la masse adhésive à une température constante jusqu'à un poste (B) d'enduction monté en combinaison avec des organes de commande (6-7) et (8-9) aptes à assurer l'entraînement linéaire du support en le maintenant à l'état rétracté, avec une tension réduite, pour assurer l'enduction de la face correspondante dudit support.

-6- Moyens selon la revendication 5, caractérisés en ce qu'à la sortie du poste d'enduction et des organes d'entraînement (6-7) et (8-9), la bande rendue adhésive coopère avec un poste de réception pour être appliquée sur un support notamment sous forme d'un ruban de papier siliconé (12).

-7- Moyens selon la revendication 5, caractérisés en ce que les organes de commande et d'entraînement (6-7) et (8-9) sont composés de deux ensembles de cylindres rotatifs montés de part et d'autres du poste d'enduction, chacun des ensembles comprenant deux cylindres superposés entre lesquels passe le support (1), les cylindres supérieurs (6) et (8) étant montés en pression sur les cylindres inférieurs (7) et (9).

-8- Moyens selon la revendication 5, caractérisés en ce que le poste d'enduction comprend des buses d'injection chauffantes (4) reliées à un organe délivreur (5) apte à distribuer la masse adhésive sous forme d'un film d'épaisseur constante, au fur et à mesure de l'avance du support (1) à l'état rétracté.

-9- Moyens selon les revendications 7 et 8, caracérisés en ce que, entre les cylindres motorisés (6-7) et (8-9) et sous l'organe distributeur (5), sont disposés des rouleaux constituant un plan d'appui horizontal pour le support (1), en vue de l'application du film de masse adhésive.

-10- Moyens selon l'une quelconque des revendications 5 à 9, caractérisés en ce que la vitesse linéaire du support est intégrée pour commander en conséquence le débit de la masse adhésive d'une manière proportionnelle.

-11- Moyens selon la revendication 6, caractérisés en ce que le ruban de papier siliconé (12) est engagé entre les cylindres (8) et (9) pour assurer l'enroulement, par friction, du support et dudit papier.

-12- Moyens selon la revendication 7, caractérisés en ce que le cylindre (9) d'enroulement du support (1) et du papier (12) est refroidi.

FIG.1

FIG.2

EP 0 336 857 A1

FIG.3

FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-A-1 491 205 (PAULIS SILK COMP.) * revendications 1,6,7,14; page 13, lignes 2-5 * --- | 1,3,5 | A 61 F 13/02 |
| A | FR-A-2 285 112 (MOLINIER S.A. et al.) * revendication 1; figures * --- | 1 | |
| A | DE-U-1 748 306 (LOHMANN KG) * revendications 1,3 * --- | 1 | |
| A | FR-A-2 146 127 (STE RHODIACETA) * revendications 1,2; figures 1,2 * ----- | 1,3,4 | |

**DOMAINES TECHNIQUES RECHERCHES (Int. Cl.5)**

A 61 F 13/00

Le présent rapport a été établi pour toutes les revendications

| Lieu de la recherche | Date d'achèvement de la recherche | Examinateur |
|---|---|---|
| BERLIN | 14-06-1989 | KANAL P K |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
......................................................................
& : membre de la même famille, document correspondant

EPO FORM 1503 03.82 (P0402)